# FASCICULE DE BREVET EUROPEEN

(11) **EP 0 423 009 B1**
(45) Date de publication et mention de la délivrance du brevet: **27.12.1995**
(21) Numéro de dépôt: 90402785.1
(22) Date de dépôt: 08.10.1990
(51) Int. Cl.: C07D 317/46

(54) **Procédé de préparation de difluoro-2,2 benzodioxole-1,3**
Verfahren zur Herstellung von 2,2-Difluoro-1,3-Benzodioxole
Process for the preparation of 2,2-difluoro-1,3-benzodioxole

(30) Priorité: 09.10.1989 FR 8913369
(43) Date de publication de la demande: 17.04.1991
(73) Titulaire: RHONE-POULENC CHIMIE, 92408 Courbevoie Cédex (FR)
(72) Inventeur: Casado, Michel, F-69360 St-Symphorien d'Ozon (FR); Crochemore, Michel, F-69630 Chaponost (FR); Langlois, Bernard, F-69006 Lyon (FR)
(74) Mandataire: Ricalens, François

(56) Documents cités:
- EP-A- 0 124 002
- DE-A- 3 642 256

## Description

La présente invention concerne un nouveau procédé de préparation du difluoro-2,2 benzodioxole-1,3 à partir du dichloro-2,2 benzodioxole-1,3.

Le brevet EP-A-0 006 978 décrit l'échange chlore-fluor dans la molécule de dichloro-2,2 benzodioxole-1,3 par le fluorure d'hydrogène liquide généralement à des températures de - 10°C à 0°C.

Les rendements obtenus sont bons, mais néanmoins on cherche encore à les améliorer. En outre, la technique de synthèse dans HF nécessite un matériel spécifique qui n'est pas disponible sur tous les sites industriels.

La présente invention se propose d'améliorer encore le rendement de la transformation du dichloro-2,2 benzodioxole-1,3 en difluoro-2,2 benzodioxole-1,3.

Plus précisément l'invention consiste en un procédé de préparation de difluoro-2,2 benzodioxole-1,3 par réaction du dichloro-2,2 benzodioxole-1,3 avec le fluorure de potassium en présence d'une quantité efficace d'un fluorure acide de potassium, de sodium, de césium, de rubidium ou d'ammonium quaternaire.

Le catalyseur utilisé est de préférence le fluorure acide de potassium KHF₂.

Il peut être généré dans le milieu réactionnel à partir du fluorure de potassium, en présence de faibles quantités d'eau. En effet cette eau, qui peut notamment provenir du fluorure de potassium lorsque celui-ci n'est pas parfaitement anhydre, réagit sur le dichloro-2,2 benzodioxole-1,3 pour former du carbonate de pyrocatéchol et génère du chlorure d'hydrogène, donnant avec le fluorure de potassium du fluorure acide de potassium et du chlorure de potassium.

Il peut être également généré dans le milieu réactionnel à partir du fluorure de potassium, en introduisant un acide fort anhydre tel que le chlorure d'hydrogène ou le bromure d'hydrogène.

Il est cependant plus commode d'introduire du fluorure acide de potassium comme catalyseur, car il est ainsi possible de contrôler la quantité mise en oeuvre. On évite également de cette façon une consommation inutile de dichloro-2,2 benzodioxole-1,3.

Mais un des avantages du procédé de l'invention est de pouvoir être réalisé même en présence de traces d'eau.

La quantité de fluorure de potassium représente au moins sensiblement la stoechiométrie nécessaire pour former le difluoro-2,2 benzodioxole-1,3.

Généralement le rapport molaire KF/dichloro-2,2 benzodioxole-1,3 est de 2 à 4 et de préférence de 2 à 2,5.

La quantité de catalyseur et plus particulièrement de KHF₂, se situe généralement entre 0,5 et 50 % en poids par rapport au poids de dichloro-2,2 benzodioxole-1,3 engagé.

De préférence le catalyseur représente par rapport au dichloro-2,2 benzodioxole-1,3 de 5 % à 20 % en poids.

La réaction s'effectue habituellement en solution.

Le solvant utilisé peut être notamment un solvant aprotique polaire.

On peut citer à titre d'exemples non limitatifs de solvants convenant tout particulièrement la tétraméthylène sulfone (ou sulfolane) et l'acétonitrile.

La concentration initiale en dichloro-2,2 benzodioxole-1,3 dans le solvant peut varier largement et n'est pas critique.

Généralement, si on l'exprime en poids de dichloro-2,2 benzodioxole-1,3 par rapport au poids total solvant-dichloro-2,2 benzodioxole-1,3, elle est de 10 % à 50 % et de préférence de 15 % à 40 %.

La température à laquelle est réalisée la réaction d'échange chlore-fluor peut également varier dans de larges limites, par exemple entre 80 et 250°C.

Afin d'avoir une cinétique suffisamment élevée tout en évitant au maximum de dégrader les réactifs, on opère de préférence entre 100°C et 200°C.

Le dichloro-2,2 benzodioxole-1,3 est un produit connu. Il peut être obtenu notamment par photochloration du méthylènedioxy-1,2 benzène à l'aide du chlore gazeux dans un hydrocarbure chloré et en présence d'un phosphate organique tel que le phosphate de tributyle.

On peut également se référer pour cette préparation à l'article du Journal of Chemical Society vol. 93, page 566 (1908).

Il peut aussi être obtenu à partir du carbonate de pyrocatéchol [Chem. Berichte vol. 96, 1382 (1963)].

Le difluoro-2,2 benzodioxole-1,3 est un composé intermédiaire qui sert notamment pour la préparation de produits pour l'agrochimie et pour la synthèse de produits pharmaceutiques.

Les exemples qui suivent illustrent l'invention.

### EXEMPLE 1

Dans un ballon tricol en verre, muni d'une agitation centrale, d'un réfrigérant ascendant et de moyens de chauffage, on charge :
- 210 g (3,61 mol) de KF anhydre (séché à 150°C sous vide)
- 23 g de KHF₂ (séché sur P₂O₅)
- 500 g de tétraméthylènesulfone (distillée sur tamis moléculaire)
- 226 g (1,18 mol) de dichloro-2,2 benzodioxole-1,3.

On chauffe sous agitation à 140°C et on suit l'évolution de la réaction par prélèvements périodiques et dosages par chromatographie en phase gazeuse (CPG).

Après 8 h, la réaction est terminée : le taux de transformation du dichloro-2,2 benzodioxole-1,3 est de 100 %.

Le mélange réactionnel est refroidi ; on rajoute 2000 g d'eau afin de solubiliser les sels et la tétraméthylènesulfone : on observe la décantation d'une phase organique constituée essentiellement par le difluoro-2,2 benzodioxole-1,3, que l'on purifie par distillation.

On obtient 155 g (0,98 mol) de difluoro-2,2 benzodioxole-1,3 pur isolé, ce qui représente un rendement (RR %) par rapport au dichloro-2,2 benzodioxole-1,3 engagé de 83 %.

### EXEMPLE 2

Dans un tube en verre de 30 cm³, muni d'un bouchon en téflon, d'une agitation magnétique et d'un système de chauffage, on charge :
- 2,95 g (0,015 mol) de dichloro-2,2 benzodioxole-1,3
- 6,5 g de tétraméthylène sulfone
- 2,95 g (0,050 mol) de KF anhydre
- 0,3 g de KHF₂.

On chauffe sous agitation à 140°C pendant 7 heures.

On dose les produits obtenus par CPG.

| | |
|---|---|
| - Taux de transformation (TT %) du dichloro-2,2 benzodioxole-1,3 | 100 % |
| - RR % en chloro-2 fluoro-2 benzodioxole-1,3 | 5 % |
| - RR % en difluoro-2,2 benzodioxole-1,3 | 95 % |

### Essai comparatif A

L'exemple 2 est répété mais en omettant le catalyseur KHF₂.

On obtient les résultats suivants :

| | |
|---|---|
| - TT % du dichloro-2,2 benzodioxole-1,3 | 93 % |
| - RR % en chloro-2 fluoro-2 benzodioxole-1,3 | 91 % |
| - RR % en difluoro-2,2 benzodioxole-1,3 | 2 % |

### EXEMPLE 3

On répète l'exemple 2 dans les mêmes conditions et avec les mêmes réactifs, mais en remplaçant KHF₂ par 0,05 g d'eau.

Après 7 heures de chauffage à 140°C, on obtient les résultats suivants :

| | |
|---|---|
| - TT % du dichloro-2,2 benzodioxole-1,3 | 100 % |
| - RR % en chloro-2 fluoro-2 benzodioxole-1,3 | 0 % |
| - RR % en difluoro-2,2 benzodioxole-1,3 | 66 % |

On dose en outre 20 % de carbonate de pyrocatéchol par rapport au dichloro-2,2 benzodioxole-1,3 de départ.

### EXEMPLE 4

On répète l'exemple 2 dans les mêmes conditions et avec les mêmes réactifs, mais en remplaçant KHF₂ par 0,1 g d'HCl gazeux.

Après 7 heures de chauffage à 140°C, on obtient les résultats suivants :

| | |
|---|---|
| - TT % du dichloro-2,2 benzodioxole-1,3 | 100 % |
| - RR % en chloro-2 fluoro-2 benzodioxole-1,3 | 1 % |
| - RR % en difluoro-2,2 benzodioxole-1,3 | 83 % |

### EXEMPLE 5

On répète l'exemple 2 dans les mêmes conditions et avec les mêmes réactifs, mais en remplaçant KHF₂ par 0,3 g de fluorure acide de benzyltriméthylammonium.

Après 7 heures de chauffage à 140°C, on obtient les résultats suivants :

| | |
|---|---|
| - TT % du dichloro-2,2 benzodioxole-1,3 | 100 % |
| - RR % en chloro-2 fluoro-2 benzodioxole-1,3 | 1 % |
| - RR % en difluoro-2,2 benzodioxole-1,3 | 81 % |

## Revendications

1. Procédé de préparation de difluoro-2,2 benzodioxole-1,3 par réaction du dichloro-2,2 benzodioxole-1,3 avec le fluorure de potassium, caractérisé en ce que l'on opère en présence d'une quantité efficace d'un fluorure acide de potassium (HF₂⁻) (K⁺), de sodium (HF₂⁻) (Na⁺), de césium (HF₂⁻) (Cs⁺), de rubidium (HF₂⁻ )(Rb⁺) ou d'ammonium quaternaire (HF₂⁻) (NH₄⁺).

2. Procédé selon la revendication 1, caractérisé en ce que le catalyseur utilisé est le fluorure acide de potassium KHF₂.

3. Procédé selon l'une des revendications 1 ou 2, caractérisé en ce que le rapport molaire KF/dichloro-2,2 benzodioxole-1,3 est de 2 à 4 et de préférence de 2 à 2,5.

4. Procédé selon l'une des revendications 1 à 3, caractérisé en ce que la quantité de catalyseur, et plus particulièrement de KHF₂, se situe entre 0,5 et 50 % en poids par rapport au poids de dichloro-2,2 benzodioxole-1,3 engagé et de préférence entre 5 % et 20 % en poids.

5. Procédé selon l'une des revendications 1 à 4, caractérisé en ce que la réaction s'effectue en solution dans un solvant aprotique polaire.

6. Procédé selon la revendication 5, caractérisé en ce que le solvant utilisé est choisi parmi la tétraméthylène sulfone (ou sulfolane) et l'acétonitrile.

7. Procédé selon l'une des revendications 1 à 6, caractérisé en ce que la concentration initiale en dichloro-2,2 benzodioxole-1,3 dans le solvant, exprimée en poids de dichloro-2,2 benzodioxole-1,3 par rapport au poids total solvant-dichloro-2,2 benzodioxole-1,3, est de 10 % à 50 % et de préférence de 15 % à 40 %.

8. Procédé selon l'une des revendications 1 à 7, caractérisé en ce que la température à laquelle est réalisée la réaction d'échange chlore-fluor se situe entre 80 et 250°C et de préférence entre 100°C et 200°C.

## Claims

1. Process for the preparation of 2,2-difluoro-1,3-benzodioxole by reaction of 2,2-dichloro-1,3-benzodioxole with potassium fluoride, characterized in that the process is performed in the presence of an effective amount of a hydrogen fluoride of potassium (HF₂⁻)(K⁺), of sodium (HF₂⁻)(Na⁺), of caesium (HF₂⁻)(Cs⁺), of rubidium (HF₂⁻)(Rb⁺) or of quaternary ammonium (HF₂⁻)(NH₄⁺).

2. Process according to claim 1, characterized in that the catalyst used is potassium hydrogen fluoride KHF₂.

3. Process according to either of claims 1 and 2, characterized in that the KF/2,2-dichloro-1,3-benzodioxole molar ratio is from 2 to 4 and preferably from 2 to 2.5.

4. Process according to one of claims 1 to 3, characterized in that the amount of catalyst, and more particularly of KHF₂, is between 0.5 and 50 % by weight relative to the weight of 2,2-dichloro-1,3-benzodioxole employed and preferably between 5 % and 20 % by weight.

5. Process according to one of claims 1 to 4, characterized in that the reaction is carried out in solution in a polar aprotic solvent.

6. Process according to claim 5, characterized in that the solvent used is chosen from tetramethylene sulphone (or sulpholane) and acetonitrile.

7. Process according to one of claims 1 to 6, characterized in that the initial concentration of 2,2-dichloro-1,3-benzodioxole in the solvent, expressed as weight of 2,2-dichloro-1,3-benzodioxole relative to the total solvent/2,2-dichloro-1,3-benzodioxole weight, is from 10 % to 50 % and preferably from 15 % to 40 %.

8. Process according to one of claims 1 to 7, characterized in that the temperature at which the chlorine/fluorine exchange reaction is carried out is between 80 and 250°C and preferably between 100°C and 200°C.

## Patentansprüche

1. Verfahren zur Herstellung von 2,2-Difluor-1,3-benzodioxol durch Reaktion von 2,2-Dichlor-1,3-benzodioxol mit Kaliumfluorid, dadurch gekennzeichnet, daß man es in Anwesenheit einer wirksamen Menge Kaliumhydrogenfluorid (K⁺)(HF₂⁻), Natriumhydrogenfluorid (Na⁺)(HF₂⁻), Cäsiumhydrogenfluorid (Cs⁺)(HF₂⁻), Rubidiumhydrogenfluorid (Rb⁺) (HF₂⁻) oder Ammoniumhydrogenfluorid (NH₄⁺)(HF₂⁻) durchführt.

2. Verfahren nach Anspruch 1, dadurch gekennzeichnet, daß der verwendete Katalysator Kaliumhydrogenfluorid KHF₂ ist.

3. Verfahren nach einem der Ansprüche 1 oder 2, dadurch gekennzeichnet, daß das Molverhältnis KF/2,2-Dichlor-1,3-benzodioxol 2 bis 4 und vorzugsweise 2 bis 2,5 beträgt.

4. Verfahren nach einem der Ansprüche 1 bis 3, dadurch gekennzeichnet, daß die Menge an Katalysator, insbesondere an KHF₂, 0,5 bis 50 Gew.-%, vorzugsweise 5 bis 20 Gew.-%, beträgt, bezogen auf das Gewicht des eingesetzten 2,2-Dichlor-1,3-benzodioxols.

5. Verfahren nach einem der Ansprüche 1 bis 4, dadurch gekennzeichnet, daß die Reaktion in Lösung in einem polaren, aprotischen Lösemittel durchgeführt wird.

6. Verfahren nach Anspruch 5, dadurch gekennzeichnet, daß das verwendete Lösemittel ausgewählt ist aus Tetramethylensulfon (Sulfolan) und Acetonitril.

7. Verfahren nach einem der Ansprüche 1 bis 6, dadurch gekennzeichnet, daß die Anfangskonzentration an 2,2-Dichlor-1,3-benzodioxol in dem Lösemittel 10 bis 50 %, vorzugsweise 15 bis 40 %, beträgt, berechnet als Gew.-% an 2,2-Dichlor-1,3-benzodioxol, bezogen auf das Gesamtgewicht Lösemittel/2,2-Dichlor-1,3-benzodioxol.

8. Verfahren nach einem der Ansprüche 1 bis 7, dadurch gekennzeichnet, daß die Temperatur, bei der die Chlor/Fluor-Austauschreaktion durchgeführt wird, 80 bis 250 °C, vorzugsweise 100 bis 200 °C, beträgt.
